# EUROPÄISCHE PATENTSCHRIFT

(11) **EP 1 318 128 B1**
(45) Veröffentlichungstag und Bekanntmachung des Hinweises auf die Patenterteilung: **18.04.2007**
(21) Anmeldenummer: 02027069.0
(22) Anmeldetag: 03.12.2002
(51) Int. Cl.: C07C 29/141, C07C 33/03

(54) **Verfahren zur selektiven Hydrierung von olefinisch ungesättigten Carbonylverbindungen**
Process for the selective hydrogenation of olefinically unsubstituted carbonyl compounds
Procédé pour l'hydrogénation de composés carbonylés oléfiniquement insaturés

(30) Priorität: 07.12.2001 DE 10160143
(43) Veröffentlichungstag der Anmeldung: 11.06.2003
(73) Patentinhaber: BASF Aktiengesellschaft, 67056 Ludwigshafen (DE)
(72) Erfinder: Göbbel, Hans-Georg, Dr., 67169 Kallstadt (DE); Gerlach, Till, Dr., 67071 Ludwigshafen (DE); Funke, Frank, Dr., 68161 Mannheim (DE); Ebel, Klaus, Dr., 68623 Lampertheim (DE); Unverricht, Signe, Dr., 68169 Mannheim (DE); Kaibel, Gerd, Dr., 68623 Lampertheim (DE)

(56) Entgegenhaltungen:
- EP-A- 0 024 651
- EP-A- 0 798 039
- US-A- 4 100 180
- SINGH U.K.,VANNICE M.A.: "Liquid-Phase Citral hydrogenation over SiO2-Supported Group VIII Metals" JOURNAL OF CATALYSIS, Bd. 199, 27. Februar 2001 (2001-02-27), Seiten 73-84, XP002253580
- BACHILLER-BOEZA B. ET AL.: "Hydrogenation of cItral on Activated Carbon and High-Surface Area Graphite-Supported Ruthenium Catalysts Modified with Iron" JOURNAL OF CATALYSIS, Bd. 204, 25. Oktober 2001 (2001-10-25), Seiten 450-459, XP002253581
- SALMI T. ET AL.: "Liquid-phase hydrogenation of citral over an immobile silica fibre catalyst" APPLIED CATALYSIS A: GENERAL, Bd. 196, 2000, Seiten 93-102, XP002253582
- PAULOSE M.M. ET AL.: "Continuous Hydrogenation of Citral to Citronellol, Geraniol and Nerol" RESEARCH AND INDUSTRY, Bd. 17, März 1972 (1972-03), Seiten 11-12, XP009015172
- NERI G. ET AL.: "Selective Hydrogenation of Citral over Pt-Sn Supported on Activated Carbon" J. CHEM. TECH. BIOTECHNOLOGY, Bd. 60, 1994, Seiten 83-88, XP000440727

## Beschreibung

Die vorliegende Erfindung betrifft ein Verfahren zur selektiven Hydrierung von α,β-ungesättigten Carbonylverbindungen zu ungesättigten Alkoholen, insbesondere von Citral zu einem Gemisch aus Geraniol und Nerol, in einem Reaktor, der eine Flüssigphase enthält, in der mindestens ein Katalysator suspendiert ist, und der gegebenenfalls zusätzlich ein Gasphase enthalten kann, wobei die Flüssigphase und gegebenenfalls die Gasphase durch eine Vorrichtung mit Öffnungen oder Kanälen in dem Reaktor geführt wird, deren hydraulischer Durchmesser 0,5 bis 20 mm beträgt, und dass suspendierte Katalysatorpartikel mit einer mittleren Körngröße von 0,0001 bis 2 mm verwendet werden.

Citral und die entsprechenden Hydroverbindungen finden als Riechstoffe Anwendung und werden darüber hinaus als Ausgangsstoffe in der Synthese von Vitaminen eingesetzt.

Aus dem Stand der Technik sind verschiedene Hydrierverfahren für α,β-ungesättigte Carbonylverbindungen bekannt. Es ist sehr schwierig, hohe Selektivitäten der entsprechenden ungesättigten Alkohole zu erhalten. Bei der Hydrierung von Citral können neben der Aldehydgruppe auch die olefinischen Doppelbindungen hydriert werden oder nur die zur Aldehydgruppe konjugierte Doppelbindung, so dass neben den ungesättigten Alkoholen Geraniol bzw. Nerol auch Nebenprodukte wie Citronellol oder Citronellal entstehen können.

US 4,100,180 beschreibt ein diskontinuierliches Verfahren zur Hydrierung von ungesättigten Aldehyden zu ungesättigten Alkoholen in Gegenwart eines PtO/Zn/Fe-Katalysators. PtO-Pulver wird mit Zn und Fe dotiert (Suspensionskatalysator). Bei der Citralhydrierung werden bei 70 % Citralumsatz 3,2 % Citronellol erhalten. Im Reaktionsaustrag befinden sich bis zu 25 ppm Feund Zn-Verbindungen. Wird der Katalysator wiederverwendet, müssen erneut geringe Mengen Fe- und Zn-Verbindung zugegeben werden.

EP 798 039 offenbart ein Verfahren zur Durchführung von katalytischen Reaktionen in einem Reaktor, der eine Flüssigphase enthält, in der mindestens ein Katalysator suspendiert ist. Häufig ist auch eine Gasphase vorhanden, aus der Reaktionsedukte in die flüssige Phase eingelöst werden. Typische Reaktionen dieser Art sind Oxidationen und Hydrierungen. Beschrieben wird die Hydrierung von Hydrodehydrolinalool zu Hydrolinalool und weiter zu Tetrahydrolinalool. Dabei wird die Dreifachbindung zunächst zur Doppelbindung und schließlich zur Einfachbindung hydriert. Eine selektive Hydrierung einer Carbonylfunktion von α,β-ungesättigten Carbonylverbindungen wird nicht beschrieben.

Diskontinuierliche Verfahren in Rührkesselreaktoren sind teil-weise sehr aufwendig, langwierig (ein Reaktionszyklus besteht aus einer Rüstzeit für Edukte und Katalysator, der Einstellung der Reaktionsbedingungen [Druck und Temperatur], der eigentlichen Reaktionszeit, gefolgt von Abkühlung und Entspannung der Reaktoren und der Abtrennung und Rückführung der Katalysatoren) und personalintensiv und eignen sich eher für die Herstellung kleiner Tonnagen. Bei großen Tonnagen führt diese Vorgehensweise zu sehr großen Reaktionsapparaten bzw. zu sehr langen Reaktionszeiten.

Der Einsatz von kontinuierlich betriebenen Festbettreaktoren umgeht den Chargenbetrieb, ist daher kostengünstiger und weniger personalintensiv, hat aber den großen Nachteil, dass für den Einsatz im Festbett speziell präparierte Katalysatoren hergestellt und eingesetzt werden müssen, die bei Verlust der Aktivität (sehr oft werden geringe Standzeiten beobachtet) nur in aufwendiger Weise ausgetauscht oder regeneriert werden können, was in der Regel mit der Abstellung der gesamten Anlage, d.h. nicht nur der Hydrierstufe, sondern auch der folgenden Aufarbeitungsstufe, verbunden ist.

Der vorliegenden Erfindung lag daher die Aufgabe zugrunde, ein robustes, kostengünstiges Verfahren zur selektiven Hydrierung der Carbonylfunktion von α,β-ungesättigten Carbonylverbindungen, insbesondere von Citral zu Geraniol und Nerol, zu entwickeln, bei dem die oben erläuterten Nachteile vermieden werden können, mit gleichzeitig erhöhten Raum-Zeit-Ausbeuten.

Die Aufgabe wurde erfindungsgemäß gelöst durch ein Verfahren zur selektiven Hydrierung von olefinisch ungesättigten Carbonylverbindungen der allgemeinen Formel I, 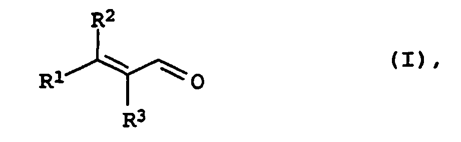 wobei
- R¹, R²: jeweils unabhängig voneinander gleich oder verschieden sein können und Wasserstoff oder einen ein- oder mehrfach ungesättigten geradkettigen oder verzweigten gegebenenfalls substituierten C₁-C₂₀-Alkylrest, einen gegebenenfalls substituierten Arylrest oder eine gegebenenfalls substituierte heterocyclische Gruppe, darstellt, und
- R³: Wasserstoff oder einen C₁-C₄-Alkylrest bedeutet
zu den entsprechenden ungesättigten Alkoholen der allgemeinen Formel II 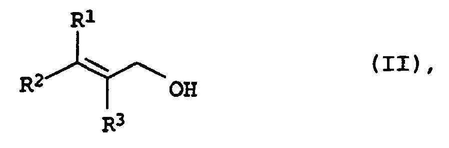
wobei R¹, R² und R³ die oben angegebene Bedeutung haben, in einem Reaktor, der eine Flüssigphase enthält, in der mindestens ein Katalysator suspendiert ist und der gegebenenfalls zusätzlich eine Gasphase enthalten kann, dadurch gekennzeichnet, dass die Flüssigphase und gegebenenfalls die Gasphase durch eine Vorrichtung mit Öffnungen oder Kanälen in dem Reaktor geführt wird, deren hydraulischer Durchmesser 0,5 bis 20 mm beträgt, und dass suspendierte Katalysatorpartikel mit einer mittleren Korngröße von 0,0001 bis 2 mm verwendet werden.

Bei der obigen Reaktion handelt es sich um die Hydrierung einer α,β-ungesättigten Carbonylverbindung, bei der überraschenderweise die Carbonylfunktion unter Erhalt der Doppelbindungen hydriert wird.

Unter einem ein oder mehrfach ungesättigten geradkettigen oder verzweigten C₁-C₁₀-Alkylrest versteht man, wenn nicht anders angegeben einen Methyl, Ethyl, Propyl, Isopropyl, n-Butyl, i-Butyl-, t-Butyl, Pentyl, Hexyl, Heptenyl, Octyl-, Nonyl-, Decyl, 1-Propenyl-, 2-Propenyl-, 2-Methyl-2-propenyl, 1-Pentenyl, 1-Methyl-2-Pentenyl-, Isopropenyl-, 1-Butenyl-, Hexenyl-, Heptenyl-, Octenyl-, Nonenyl-, oder einen Decenylrest bzw. die den weiter unten aufgeführten Verbindungen entsprechenden Reste.

Unter einem C₁-C₄-Alkylrest versteht man, wenn nicht anders angegeben einen Methyl-, Ethyl-, Propyl-, i-Propyl-, Butyloder t-Butylrest.

Unter einem Arylrest versteht man einen Benzyl, Phenyl- oder Naphthylrest.

Unter einer heterocyclischen Gruppe versteht man beispielsweise einen Pyridin-, Pyrimidin, Pyridazin, Pyrazin-, Piperazin-, Imidazol-, Furan, Oxazol, Isothiazol, Isoxazol, 1,2,3-Triazoloder 1,2,4-Triazol, Thiazol-, Thiophen- oder Indolring.

Substituenten bedeuten Methyl-, Ethyl-, Propyl-, i-Propyl-, Butyl-, t-Butyl-, Fluor-, Chlor-, Brom-, Iod-, Nitro- oder Aminoreste.

Bevorzugt eingesetzte olefinisch ungesättigte Carbonylverbindungen der Formel I sind α,β-ungesättigte Carbonylverbindungen wie beispielsweise Acrolein, Methacrolein, Crotonaldehyd, Prenal, Farnesal oder Citral, insbesonders bevorzugt Citral.

In einer bevorzugten Ausgestaltung des Verfahrens wird Citral zu einem Gemisch von Geraniol und Nerol umgesetzt.

Die Hydrierung wird in einem Reaktor gemäß EP 798 039 durchgeführt, in dem die Flüssig- und die Gasphase durch eine Vorrichtung mit Öffnungen oder Kanälen mit einem hydraulischen Durchmesser von 0,5 bis 20 mm, vorzugsweise 1 bis 10 mm, besonders bevorzugt 1 bis 3 mm, durchgeleitet wird. Der hydraulische Durchmesser ist als Quotient des 4-fachen Querschnitts der Öffnung und deren Umfang definiert.

Die Vorrichtung mit Öffnungen oder Kanälen zur Durchleitung des Reaktionsmediums kann aus einer Schüttung, einem Gestrick, einer offenzelligen Schaumstruktur, vorzugsweise aus Kunststoff (z.B. Polyurethan oder Melaminharze) oder Keramik, oder einem Packungselement, wie es grundsätzlich, d.h. seiner geometrischen Form nach, bereits aus der Destillations- und Extraktionstechnik bekannt ist, bestehen.

Derartige Packungselemente, die den Vorteil eines geringen Druckverlustes bieten, sind z.B. Drahtgewebepackungen. Für Zwecke der vorliegenden Erfindung haben die Packungen jedoch grundsätzlich einen wesentlich, regelmäßig um den Faktor 2 bis 10 kleineren hydraulischen Durchmesser als vergleichbare Einbauten im Bereich der Destillations- und Extraktionstechnik.

Drahtgewebepackungen sind besonders vorteilhaft. Das beruht darauf, dass ein Teil der suspendierten Partikel von dem Gewebe zurückgehalten werden und sich die Relativgeschwindgkeit Feststoff/Fluid deutlich verbessert. Statt Gewebepackungen können aber im Rahmen der vorliegenden Erfindung auch Packungen aus anderen gewebten, gewirkten oder gefilzten flüssigkeitsdurchlässigen Materialien verwendet werden. Bei weiteren geeigneten Packungen werden ebene Bleche, bevorzugt ohne Perforation oder andere größere Öffnungen, eingesetzt, beispielsweise entsprechend den Bauarten Montz B1 oder Sulzer Mellapak. Vorteilhaft sind auch Packungen aus Streckmetall, wie z.B. Packungen des Typs Montz BSH. Auch dabei müssen Öffnungen, wie etwa Perforationen, entsprechend klein gehalten werden. Entscheidend für die Eignung einer Packung im Rahmen der vorliegenden Erfindung ist nicht deren Geometrie, sondern die für die Stromführung entstehenden Öffnungsgrößen bzw. Kanalbreiten in der Packung.

Suspensionsreaktoren benötigen die Zufuhr mechanischer Energie, die beispielsweise durch Rührwerke, Düsen oder aufsteigende Gasblasen eingebracht wird, um die Feststoffteilchen zu suspendieren. Eine Erhöhung der mechanischen Energiezufuhr über den zur Suspendierung erforderlichen Betrag hinaus führt aber bei Suspensionsreaktoren ohne Einbauten zu keiner nennenswerten Verbesserung des Stoffübergangs zwischen der Flüssigkeit und den suspendierten Feststoffteilchen, da die erzielbare Relativgeschwindigkeit die Sedimentationsgeschwindigkeit nur unwesentlich übertrifft.

Um diese Relativgeschwindigkeit zu erhöhen, wurde die Verwendung von Katalysatorpartikeln mit größeren Korngrößen (1 bis 10 mm), insbesondere in Fließ- und Wirbelschichtverfahren, vorgeschlagen. Diese größeren Teilchen besitzen gegenüber der umgebenden Flüssigkeit zwar die gewünschte Relativgeschwindigkeit, ihre geringere volumenbezogene Oberfläche begrenzt aber andererseits wiederum den Stoffumsatz. Beide Effekte kompensieren sich häufig, so dass das Problem der Erhöhung des Stofftransportes nicht gelöst wird.

Hohe Relativgeschwindigkeiten können lediglich bei Aufgabe der Suspensionsfahrweise und Nutzung der Festbettreaktoren erzielt werden, wobei der oben erwähnte Nachteil der geringen volumenbezogenen Katalysatoroberfläche noch verstärkt wird. Statt einer Schüttung des Katalysators in verstrangter oder tablettierter Form können auch Bleche oder Gewebe mit Katalysatormaterial beschichtet werden, die von der flüssigen bzw. von der gasförmigen Phase angeströmt werden. Ein Nachteil dieser Reaktoren besteht allerdings darin, dass das Katalysatormaterial nur durch den kompletten Ersatz der beschichteten Bauteile erneuert werden kann, wenn eine Kontaminierung des Katalysatormaterials durch unreine Reaktionsprodukte eintritt. Das ist aufwendig und führt, wie auch im Falle der Verwendung von Festbettreaktoren, zu aufwendigen Vorsichtsmaßnahmen, wie etwa der Hochreinigung der Ausgangsstoffe.

Im Unterschied zum Stand der Technik für die Hydrierung von α,β-ungesättigten Carbonylverbindung, insbesondere Citral, wird in der erfindungsgemäßen Hydrierung durch die Verwendung von Einbauten in den Blasensäulenreaktoren eine erhöhte Differenz in der Bewegung (hohe Relativgeschwindigkeit) der Katalysatorteilchen gegenüber der Flüssigphase im Reaktionsteil erzeugt, weil die Partikel in den engen Öffnungen und Kanälen gegenüber der umgebenden Flüssigkeit stärker zurückgehalten werden. Bei dem erfindungsgemäßen Verfahren können zur Suspension handelsübliche Katalysatorteilchen mit einer mittleren Korngröße von 0,0001 bis 2 mm, bevorzugt von 0,005 bis 1 mm, besonders bevorzugt von 0,001 bis 0,1 mm, verwendet werden. Diese Partikel führen mit ihrer hohen volumenbezogenen Oberfläche zu guten Resultaten. Im Ergebnis können deutlich höhere Raum-Zeit-Ausbeuten erzielt werden.

Bei Anwendung der erfindungsgemäßen Hydrierung werden somit hohe Relativgeschwindigkeiten der Flüssigphase gegenüber den Katalysatorteilchen, bei gleichzeitiger Verwendung von Katalysatorteilchen mit einer hohen volumenbezogenen Oberfläche, erreicht.

Die Hydrierung wird nach dem erfindungsgemäßen Verfahren in einem Reaktor mit einer der oben beschriebenen Einbauten in Gegenwart eines Suspensionskatalysators und Wasserstoff bei einem Druck zwischen 1 und 100 bar, bevorzugt 1 und 60 bar, besonders bevorzugt 1 und 50 bar, durchgeführt. Die Reaktionstemperaturen liegen zwischen 40 und 120°C, bevorzugt zwischen 60 und 100°C, besonders bevorzugt zwischen 70 und 90°C.

Nach dem erfindungsgemäßen Verfahren wird beispielsweise Citral nicht in reiner Form, sondern als aminhaltige alkoholische Lösung eingesetzt. Als Alkohole eignen sich bevorzugt C₁- bis C₄-Alkhole, als Aminkomponenten tertiäre C₁- bis C₄ Amine. Die Konzentration des Citrals beträgt in der Lösung bevorzugt 50 bis 90 Gew.-%, besonders bevorzugt 60 bis 80 Gew.-%, die des Alkohols 40 bis 5 Gew.-%, bevorzugt 20 bis 35 %, die des tertiären Amins 1 bis 15 Gew.-%, bevorzugt 1 bis 8 Gew.-%.

Als Einbauten im Hydrierreaktor werden die bereits zuvor aufgeführten Gewebepackungen oder Blechpackungen verwendet. Reaktionsgemisch, Katalysator und Wasserstoff werden mit hoher Geschwindigkeit im Kreis durch den Reaktor gepumpt. Die Querschnittsflächenbelastung von Gas- und Flüssigphase liegen hierbei über 100 m³/m²h. Die Gasphase wird mittels einer Injektordüse inniglich mit der Flüssigphase durchmischt.

Für die Hydrierung kann ein kommerziell verfügbarer Suspensionskatalysator verwendet werden, der als Aktivkomponente mindestens Ruthenium enthält. Neben Ruthenium kann der Katalysator auch weitere Aktivkomponenten, wie beispielsweise Eisen, enthalten. Der Katalysator kann in metallischer und/oder oxidischer Form unter anderem auf Trägermaterialien eingesetzt werden. Als Trägermaterialien eignen sich beispielsweise SiO₂, TiO₂, ZrO₂, Al₂O₃ oder Kohlenstoff wie Graphite, Ruße oder Aktivkohle, insbesondere bevorzugt wird Aktivkohle. Der Gehalt an Ruthenium liegt zwischen 0,1 und 10 Gew.-%, der Gehalt an Eisen liegt zwischen 0,1 und 5 Gew.-%, bevorzugt zwischen 0,5 und 1,5 Gew.-%.

Die erfindungsgemäße Hydrierung kann sowohl kontinuierlich als auch diskontinuierlich erfolgen, bevorzugt verläuft sie jedoch kontinuierlich.

Die erfindungsgemäße Hydrierung, insbesondere zur Herstellung von Geraniol und Nerol zeichnet sich dadurch aus, dass die Flüssigkeit mit einer Leerrohrgeschwindigkeit von etwa 50 bis 300 m³/m²h, vorzugsweise von 100 bis 250 m³/m²h, durch die oben beschriebene Vorrichtung mit Öffnungen und Kanälen geführt wird. Bei gleichzeitiger Anwesenheit der Gasphase beträgt deren Leerrohrgeschwindigkeit vorzugsweise 50 bis 300 m³/m²h, besonders bevorzugt 100 bis 250 m³/m²h.

Die erfindungsgemäße Hydrierung kann in verschiedenen Reaktorbauformen, wie Strahldüsenreaktoren, Blasensäulen oder Rohrbündelreaktoren, durchgeführt werden. Die oben aufgeführten Einbauten füllen vorzugsweise aber nicht notwendigerweise den gesamten Reaktor aus. Der erfindungsgemäße Reaktor ist vorzugsweise eine vertikal angeordnete Blasensäule, die bei Anwesenheit einer Gasphase bevorzugt im Gleichstrom von unten nach oben durchströmt wird. Ein anderer bevorzugter Reaktor ist ein heiz- und kühlbarer Rohrbündelreaktor, bei dem die erfindungsgemäßen Einbauten in den einzelnen Rohren untergebracht sind. Das suspendierte Katalysatormaterial kann mit Hilfe gebräuchlicher Techniken eingebracht und wieder abgetrennt werden (Sedimentation, Zentrifugation, Kuchenfiltration, Querstromfiltration).

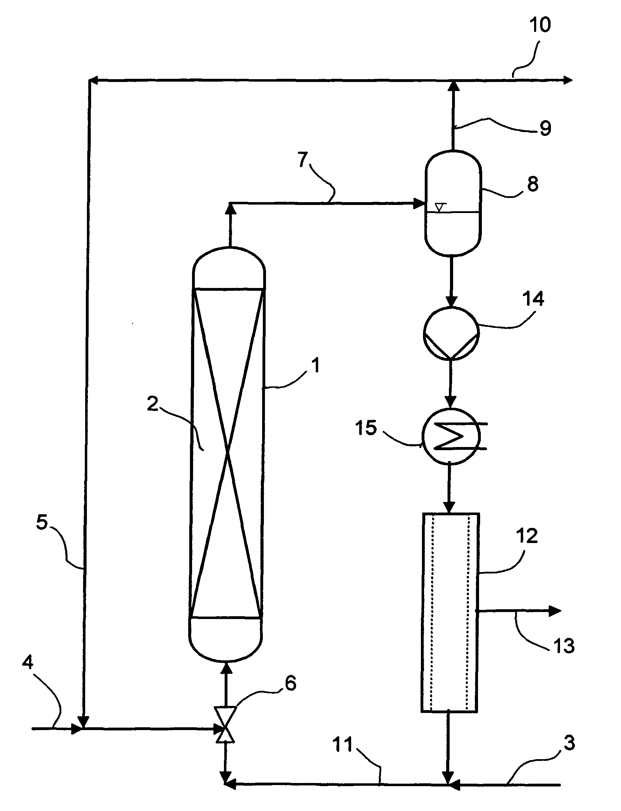

Beispielhaft wird ein Reaktor zur Hydrierung einer citralhaltigen Lösung zu einem Gemisch aus Geraniol und Nerol mit einem Ru/Fe-Kohle Suspensionskatalysator gemäß der vorliegenden Erfindung anhand der Figur 1 im Detail beschrieben. Figur 1 zeigt beispielhaft den Versuchsaufbau eines kontinuierlich betriebenen Reaktors (Blasensäule) 1 mit Packung 2, die über die Leitungen 3 mit Flüssigkeit und über die Leitung 4 mit Frischgas gespeist wird. Das Kreisgas 5 wird mittels der Mischdüse 6 mit Frischgas und der durch die Pumpe 14 im Kreis geführten Suspension 11 eingemischt. Der Reaktoraustrag wird über die Leitung 7 in das Abscheidegefäß 8 gefahren, in dem die Gasphase abgeschieden und über Leitung 9 abgeführt wird. Von dieser Gasmenge wird zur Begrenzung der Aufpegelung von gasförmigen Verunreinigungen ein Teilstrom über die Leitung 10 entnommen und die verbleibende Restmenge über die Leitung 5 in den Reaktor geführt. Der suspendierte Katalysator verbleibt im Reaktorsystem, indem er über einen Querstromfilter 12 zurückgehalten und nur katalysatorfreie Flüssigphase über die Leitung 13 austritt und entnommen wird. Über den Wärmetauscher 15 kann die Temperatur im Reaktorsystem gezielt eingestellt werden.

### Beispiel 1

Die Reaktion wurde in einer mit einer Gewebepackung bestückten Blasensäule (3000 mm Länge, 27,3 mm Durchmesser) entsprechend der vorliegenden Erfindung durchgeführt. Der Versuchsaufbau entsprach Figur 1. Die Geometrie der Packung entsprach einer handelsüblichen Gewebepackung vom Typ Montz A1 1200. Die volumenbezogene Oberfläche der Packung beträgt 1200 m²/m³, wobei sich diese Angabe nur auf die Oberfläche der Gewebe bezieht. Die Flüssigkeit mit dem suspendierten Katalysator und das Gas wurden mit einer Leerrohrgeschwindigkeit von 200 m³/m²h von unten in den gepackten Reaktor eingebracht.

Die Reaktion erfolgte kontinuierlich unter einem Wasserstoffdruck von 22 bar und einer Temperatur von 80°C. Als Katalysator wurde ein Ru/Fe-Kohle Suspensionskatalysator mit einem Gehalt von 5 % Ruthenium und 1 % Eisen auf Aktivkohle verwendet, der eine mittlere Korngröße um 50 µm aufwies.

Als Zulauf für die gepackte Blasensäule diente eine Lösung bestehend aus 70 Gew.-% Citral, 27 Gew.-% Methanol, 3 Gew.-% Trimethylamin.

Der Umsatz betrug 90 % bei einer Selektivität von > 94 % für Geraniol und Nerol und einer Citronellolselektivität von nur 1,2 %. Die Katalysatorbelastung betrug 3000 g_{Citral}/(kg_{Kat}*h) , die Raumzeitausbeute 230 kg_{Citral}/(m³h).

### Beispiel 2

Die Reaktion wurde in einer mit einer Gewebepackung bestückten Blasensäule (3000 mm Länge, 27,3 mm Durchmesser) entsprechend der vorliegenden Erfindung durchgeführt. Der Versuchsaufbau entsprach Figur 1. Die Geometrie der Packung entsprach einer handelsüblichen Gewebepackung vom Typ Montz A1 1200. Die volumenbezogene Oberfläche der Packung beträgt 1200 m²/m³, wobei sich diese Angabe nur auf die Oberfläche der Gewebe bezieht. Die Flüssigkeit mit dem suspendierten Katalysator und das Gas wurden mit einer Leerrohrgeschwindigkeit von 200 m³/m²h von unten in den gepackten Reaktor eingebracht.

Die Reaktion erfolgte kontinuierlich unter einem Wasserstoffdruck von 30 bar und einer Temperatur von 80°C. Als Katalysator wurde ein Ru/Fe-Kohle Suspensionskatalysator mit einem Gehalt von 5 % Ruthenium und 1 % Eisen auf Aktivkohle verwendet, der eine mittlerer Korngröße um 50 µm aufwies.

Als Zulauf für die gepackte Blasensäule diente eine Lösung bestehend aus 70 Gew.-% Citral, 27 Gew.-% Methanol, 3 Gew.-% Trimethylamin.

Der Umsatz betrug 96 % bei einer Selektivität von > 92 % für Geraniol u. Nerol und einer Citronellolselektivität von 4 %. Die Katalysatorbelastung betrug 2100 g_{Citral}/(kg_{Kat}*h) , die Raumzeitausbeute 230 kg_{Citral}/(m³h) .

## Patentansprüche

1. Verfahren zur selektiven Hydrierung von α,β-ungesättigten Carbonylverbindungen der allgemeinen Formel I, 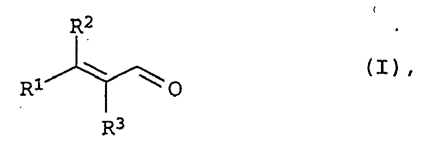 wobei
R¹, R² jeweils unabhängig voneinander gleich oder verschieden sein können und Wasserstoff oder einen ein- oder mehrfach ungesättigten geradkettigen oder verzweigten gegebenenfalls substituierten C₁-C₂₀-Alkylrest, einen gegebenenfalls substituierten Arylrest oder eine gegebenenfalls substituierte heterocyclische Gruppe darstellen, worin, sofern vorhanden, die Substituenten Methyl-, Ethyl-, Propyl-, i-Propyl-, Butyl-, t-Butyl-, Fluor-, Chlor-, Brom-, Jod-, Nitro- oder Aminoreste bedeuten, und
R³ Wasserstoff oder einen C₁-C₄-Alkylrest bedeutet
zu den entsprechenden ungesättigten Alkoholen der allgemeinen Formel II 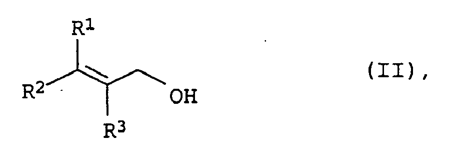 wobei R¹, R² und R³ die oben angegebene Bedeutung haben, in einem Reaktor, der eine Flüssigphase enthält, in der mindestens ein Katalysator suspendiert ist und der gegebenenfalls zusätzlich eine Gasphase enthalten kann, **dadurch gekennzeichnet, dass** die Flüssigphase und gegebenenfalls die Gasphase durch eine Vorrichtung mit Öffnungen oder Kanälen in dem Reaktor geführt wird, deren hydraulischer Durchmesser 0,5 bis 20 mm beträgt, und dass suspendierte Katalysatorpartikel mit einer mittleren Korngröße von 0,0001 bis 2 mm verwendet werden.

2. Verfahren gemäß Anspruch 1, **dadurch gekennzeichnet, dass** Citral zu einem Gemisch von Geraniol und Nerol hydriert wird.

3. Verfahren gemäß einem der Ansprüche 1 bis 2, **dadurch gekennzeichnet, dass** als Vorrichtung mit Öffnungen oder Kanälen eine Schüttung, ein Gestrick, eine offenzellige Schaumstruktur oder ein Packungselement, wie es grundsätzlich aus der Destillations- oder Extraktionstechnik bekannt ist, verwendet wird.

4. Verfahren gemäß einem der Ansprüche 1 bis 3, **dadurch gekennzeichnet, dass** die Flüssigphase und gegebenenfalls die Gasphase zumindest teilweise durch Öffnungen oder Kanäle geführt wird, deren Wandmaterialien Oberflächenrauhigkeiten im Bereich des 0,1 bis 10-fachen der mittleren Korngröße der suspendierten Katalysatorteilchen aufweisen.

5. Verfahren gemäß einem der Ansprüche 1 bis 4, **dadurch gekennzeichnet, dass** die Flüssigphase mit einer Leerrohrgeschwindigkeit von 50 bis 300 m³/m²h, eine eventuell gleichzeitig anwesende Gasphase mit einer Leerrohrgeschwindigkeit von 5 bis 300 m³/m²h durch die Vorrichtung mit Öffnungen oder Kanälen geführt wird.

6. Verfahren gemäß einem der Ansprüche 1 bis 5, **dadurch gekennzeichnet, dass** das Citral als aminhaltige, alkoholische Lösung eingesetzt wird.

7. Verfahren gemäß einem der Ansprüche 1 bis 6, **dadurch gekennzeichnet, dass** die Konzentration des Citrals in der Lösung 50 bis 90 Gew.-% beträgt.

8. Verfahren gemäß einem der Ansprüche 1 bis 7, **dadurch gekennzeichnet, dass** die Konzentration des Alkohols in der Lösung 40 bis 5 Gew.-% beträgt.

9. Verfahren gemäß einem der Ansprüche 1 bis 8, **dadurch gekennzeichnet, dass** die Konzentration des Amins in der Lösung 1 bis 15 Gew.-% beträgt.

10. Verfahren gemäß einem der Ansprüche 1 bis 9, **dadurch gekennzeichnet, dass** der Reaktor eine Blasensäule ist.

## Claims

1. A process for selective hydrogenation of α,β-unsaturated carbonyl compounds of the general formula I 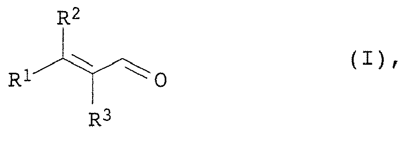 where
R¹ and R² are identical or different and are each independently hydrogen or a singly or multiply unsaturated straight chain or branched substituted or unsubstituted C₁-C₂₀-alkyl radical, an unsubstituted or substituted aryl radical or an unsubstituted or substituted heterocyclic group, in which, where available, the substituents are methyl, ethyl, propyl, i-propyl, butyl, t-butyl, fluorine, chlorine, bromine, iodine, nitro or amino radicals, and
R³ is hydrogen or C₁-C₄-alkyl,
together with corresponding unsaturated alcohols of general formula II 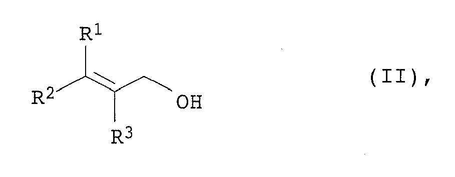 where R¹, R² and R³ are each as defined above, in a reactor comprising, in the absence or presence of a gas phase, a liquid phase holding at least one catalyst in suspension, which comprises passing the liquid phase and, if appropriate, the gas phase through a device in the reactor having openings or channels whose hydraulic diameter is in the range from 0.5 to 20 mm, and using suspended catalyst particles with an average particle size of from 0.0001 to 2 mm.

2. The process according to claim 1, wherein citral is hydrogenated to give a mixture of geraniol and nerol.

3. The process according to either of claims 1 and 2, wherein the device having openings or channels is a bed, a drawn-loop knit, an open-celled foam structure or a packing element as known in principle from distillation or extraction technology.

4. The process according to any of claims 1 to 3, wherein the liquid phase and, if appropriate, the gas phase are at least partly passed through openings or channels whose wall materials have surface roughness in the range from 0.1 to 10 times the average particle size of the suspended catalyst particles.

5. The process according to any of claims 1 to 4, wherein the liquid phase is passed through the device having openings or channels at a superficial velocity in the range from 50 to 300 m³/m²h, and any gas phase present is passed through at a superficial velocity in the range from 5 to 300 m³/m²h.

6. The process according to any of claims 1 to 5, wherein the citral is used as an aminic alcoholic solution.

7. The process according to any of claims 1 to 6, wherein the concentration of citral in the solution is in the range from 50 to 90% by weight.

8. The process according to any of claims 1 to 7, wherein the concentration of alcohol in the solution is in the range from 40 to 5% by weight.

9. The process according to any of claims 1 to 8, wherein the concentration of amine in the solution is in the range from 1 to 15% by weight.

10. The process according to any of claims 1 to 9, wherein the reactor is a bubble column.

## Revendications

1. Procédé d'hydrogénation sélective de composés carbonyle α,β-insaturés de la formule générale I : 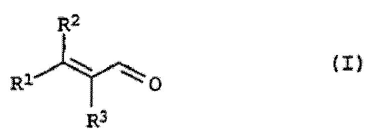 dans laquelle
R¹, R² peuvent indépendament l'un de l'autre être identiques ou différents et représenter de l'hydrogène ou un radical alkyle en C₁-C₂₀ éventuellement substitué, insaturé à une ou plusieurs reprises, à chaîne linéaire ou ramifiée, un radical aryle éventuellement substitué ou un groupe hétérocyclique éventuellement substitué, les substituants, dans la mesure où ils sont présents, représentant des radicaux méthyle, éthyle, propyle, i-propyle, butyle, t-butyle, fluoro, chloro, bromo, iodo, nitro ou amino, et
R³ représente de l'hydrogène ou un radical alkyle en C₁-C₄,
en les alcools insaturés correspondants de la formule générale II : 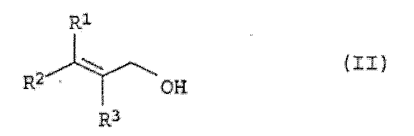 dans laquelle R¹, R² et R³ ont la signification indiquée ci-dessus, dans un réacteur qui contient une phase liquide dans laquelle au moins un catalyseur est mis en suspension et qui peut éventuellement contenir en supplément une phase gazeuse, **caractérisé en ce que** la phase liquide et éventuellement la phase gazeuse sont conduites dans le réacteur à travers un dispositif présentant des orifices ou des canaux dont le diamètre hydraulique est de 0,5 à 20 mm, et **en ce que** des particules de catalyseur en suspension présentant une taille moyenne de grain de 0,0001 à 2 mm sont utilisées.

2. Procédé suivant la revendication 1, **caractérisé en ce qu'**on hydrogène du citral en un mélange de géraniol et de nérol.

3. Procédé suivant l'une des revendications 1 et 2, **caractérisé en ce que**, comme dispositif à orifices ou canaux, on utilise un garnissage, un produit tricoté, une structure mousse à pores ouverts ou un élément de paquet, comme c'est connu principalement dans la technique de distillation ou d'extraction.

4. Procédé suivant l'une des revendications 1 à 3, **caractérisé en ce que** la phase liquide et éventuellement la phase gazeuse sont conduites au moins partiellement à travers des orifices ou des canaux dont les matériaux de paroi présentent des rugosités de surface de l'ordre de 0,1 à 10 fois la taille de grain moyenne des particules de catalyseur en suspension.

5. Procédé suivant l'une des revendications 1 à 4, **caractérisé en ce que** la phase liquide est conduite à travers le dispositif à orifices ou canaux à une vitesse dans un tube vide de 50 à 300 m³/m²h, une phase gazeuse éventuellement simultanément présente une vitesse dans un tube vide de 5 à 300 m³/m²h.

6. Procédé suivant l'une des revendications 1 à 5, **caractérisé en ce que** le citral est mis en oeuvre sous la forme d'une solution alcoolique, contenant de l'amine.

7. Procédé suivant l'une des revendications 1 à 6, **caractérisé en ce que** la concentration du citral dans la solution est de 50 à 90 % en poids.

8. Procédé suivant l'une des revendications 1 à 7, **caractérisé en ce que** la concentration de l'alcool dans la solution est de 40 à 5 % en poids.

9. Procédé suivant l'une des revendications 1 à 8, **caractérisé en ce que** la concentration de l'amine dans la solution est de 1 à 15 % en poids.

10. Procédé suivant l'une des revendications 1 à 9, **caractérisé en ce que** le réacteur est une colonne à bulles.
